# EUROPEAN PATENT APPLICATION

(11) **EP 1 836 991 A1**
(43) Date of publication of application: **26.09.2007**
(21) Application number: 07005210.5
(22) Date of filing: 14.03.2007
(51) Int. Cl.: A61C 8/00

(54) **A device for packaging and handling an Endo-osseous implant**

(30) Priority: 21.03.2006 IT MI20060512
(71) Applicant: Galvan, Mauro, 20070 Cerro al Lambro (Milano) (IT); Dell'Aquila, Luca, 26855 Lodi Vecchio (LODI) (IT)
(72) Inventor: Galvan, Mauro, 20070 Cerro al Lambro (Milano) (IT); Dell'Aquila, Luca, 26855 Lodi Vecchio (LODI) (IT)
(74) Representative: Cicogna, Franco

(57) **Abstract**

A device (1) for packaging and handling an endo-osseous implant (3) comprises a gripping and sterile blister-pack transport system (4) for gripping and transporting the implant (3) in a sterile blister pack (4) to a surgical operation situ, in a ready for use condition, the system comprising a gripper-ring nut assembly (2) bearing the implant (3), screwing on and releasing said implant (3), to provide a preset and automatic surgical stop of said implant (3).

## Description

### BACKGROUND OF THE INVENTION

The present invention relates to a device for packaging and handling an endo-osseous implant.

As is known, in modern prosthesis implanting procedures is very important to simplify the implant methods and instruments, to reduce, on a side, surgical intrusions and, on the other side, also reduce and simplify the surgical operations and tool assemblies as necessary for implanting the prosthesis.

For example, for quickly and properly implanting endo-osseous prostheses, operating recessed head stem tools and torque wrench instruments , to be either manually or automatically driven, are frequently used.

In this respect, to speed up and simplify said prosthesis implant surgical operations, the use of surgical tool operating micromotors has been suggested.

However, a micromotor system has the drawback that it is difficult to properly stop, at a proper time, the micromotor and tool driven thereby.

Some operators have been proposed to disengage the implant from the operating tool handle by breaking away it along a weakened line or portion.

However, this approach has the drawback that the bone being processed has a variable and not constant bone density.

Actually, in a high density bone (of the D1 class), a breakage of the stem-implant assembly can accidentally occur before ending the engagement operation of the implant, thereby causing the implant-prosthesis assembly to abruptly.

On the contrary, in a low density or soft bone (of the D4 class), the tool stem or handle and implant sometimes cannot be broken away from one another, with a consequent dangerous excessive penetration of the implant body into the bone.

### SUMMARY OF THE INVENTION

Accordingly, the aim of the present invention is to provide a device for packaging and handling an endo-osseous implant, adapted to overcome the above mentioned drawbacks of the prior art.

Within the scope of the above mentioned aim, a main object of the invention is to provide such a device allowing the implant to be easily gripped and transported in a sterile blister pack, to be sent, in a ready for use condition to a desired surgical operation place.

Another object of the invention is to provide such a device allowing a surgical operator to easily remove, by an electric handle fitting, the implant from its sterile blister pack, thereby reducing to a minimum the implant processing time.

Yet another object of the present invention is to provide such a device allowing the implant body to be mechanically threaded or screwed on by a micromotor.

Yet another object of the present invention is to provide such a device allowing the implant bearing-threading assembly to be automatically disengaged from the implant after having achieved a target and proper surgical operation depth in the bone.

According to one aspect of the present invention, the above mentioned aim and objects, as well as yet other objects, which will become more apparent hereinafter, are achieved by a device for packaging and handling an endo-osseous implant according to claim 1.

### BRIEF DESCRIPTION OF THE DRAWINGS

Further characteristics and advantages of the present invention will become more apparent hereinafter from the following disclosure of a preferred, though not exclusive, embodiment of the invention which is illustrated, by way of an indicative, but not limitative, example in the accompanying drawings, where:

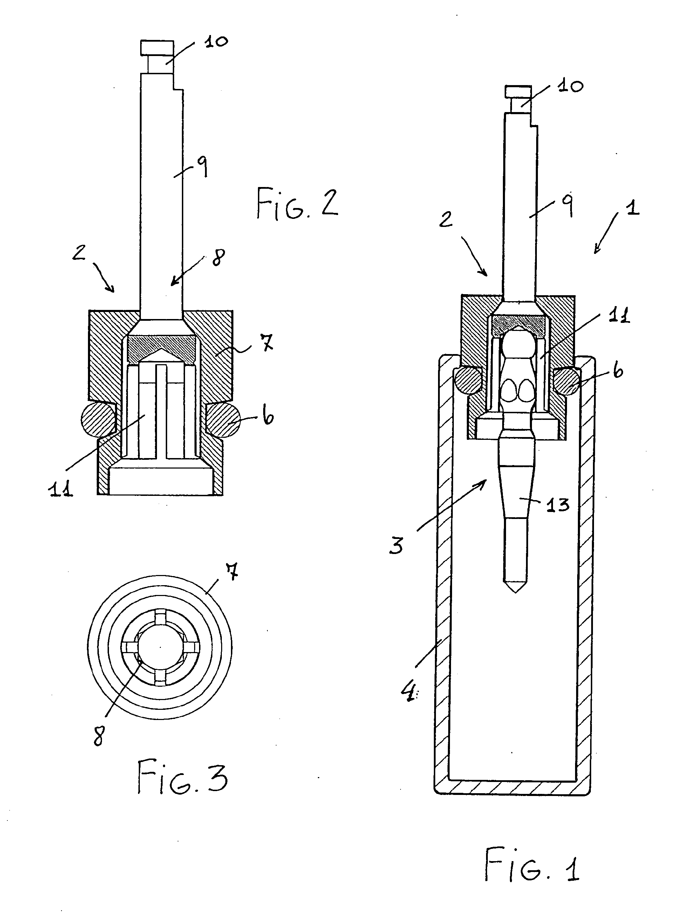

Figure 1 is an elevation longitudinally cross-sectioned view showing a device for packaging and applying an endo-osseous implant, according to the present invention;
Figure 2 is a further longitudinally cross-sectioned elevation view of the ring-nut and gripper assembly included in the device according to the invention;
Figure 3 is a top plan view of the ring-nut and gripper assembly;

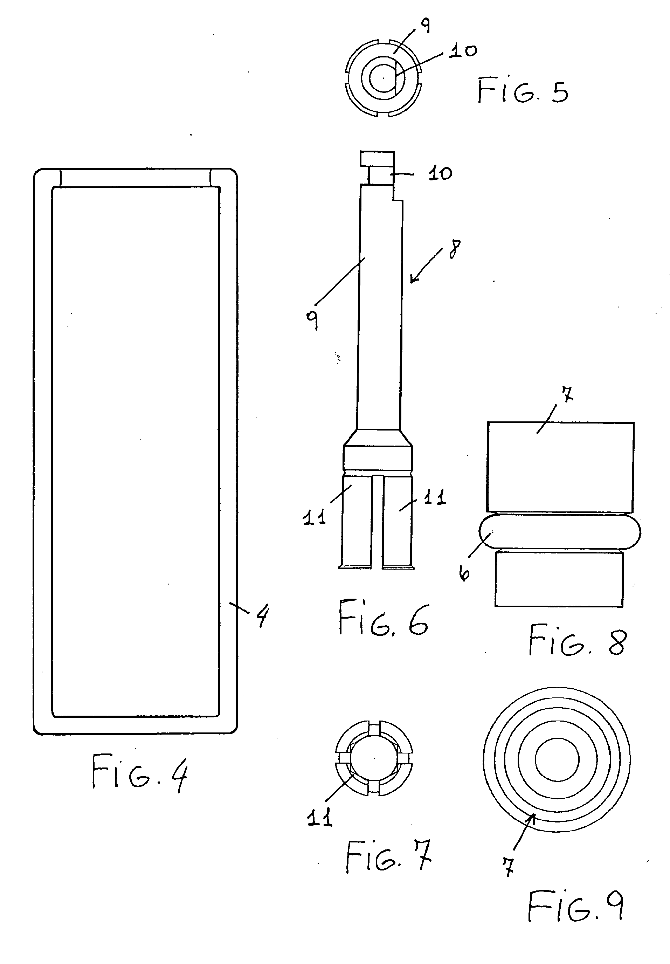

Figure 4 is an elevation view of a blister pack designed for holding in a sterile condition the device;
Figure 5 is a top plan view of the gripper;
Figure 6 is an elevation view of the gripper;
Figure 7 is a bottom view of the gripper;
Figure 8 is an elevation view of the ring-nut;
Figure 9 is a top plan view of the ring-nut;
Figure 10 is a partially cross-sectioned elevation view schematically showing a mode of operation of the device according to the present invention, in performing a threading and drilling operation;
Figure 11 is a further schematic view similar to figure 10, but showing an automatic preset disengagement operation for disengaging the ring-nut from the gripper; and
Figure 12 is a further schematic view, similar to figure 11, but showing a removal operation for removing the ring-nut and gripper assembly after having engaged the osseous implant in a proper surgical recess.

### DESCRIPTION OF THE PREFERRED EMBODIMENTS

With reference to the number references of the above mentioned figures, the device for packaging and applying an endo-osseous implant, according to the present invention, and which has been generally indicated by the reference number 1, comprises a gripper and ring-nut assembly 2 supporting an endo-osseous implant 3 arranged in a blister pack 4, made rigid with the gripper and ring-nut 2 assembly.

More specifically, the blister pack 4 substantially comprises a cylinder element, preferably made of a plastics material, closed at one end portion thereof and including, at the other end portion thereof, an opening 5 for allowing a system comprising the gripper and ring-nut 2 assembly and the implant 3 made rigid therewith to be easily and quickly introduced into said cylinder element.

More specifically, the ring-nut and gripper assembly 2 is tightly coupled to the blister pack 4 by sealing means comprising, in this embodiment, an annular gasket, such as an O-ring 6, associated with the ring-nut 7 of the ring-nut and gripper assembly 2.

The ring-nut 7 comprises an outer annular recess, for engaging therein the gasket 6, and a further inner axial recess for receiving a portion of the gripper 8 forming a part of the gripper 8 - ring nut 7 assembly 2.

As shown, the gripper 8 substantially comprises a gripper stem 9 including an end-piece 10 and rigidly coupled to a gripper portion comprising a plurality of axially extending flaps or legs 11.

The axial legs 11 are designed for engaging the stem 12 of the endo-osseous implant 3 including, in a per se known manner, a screw portion 13.

The above disclosed device operates as follows.

At first, a surgical operator removes, by an electric operating handle, the implant from its sterile blister pack, through the gripper and ring-nut assembly.

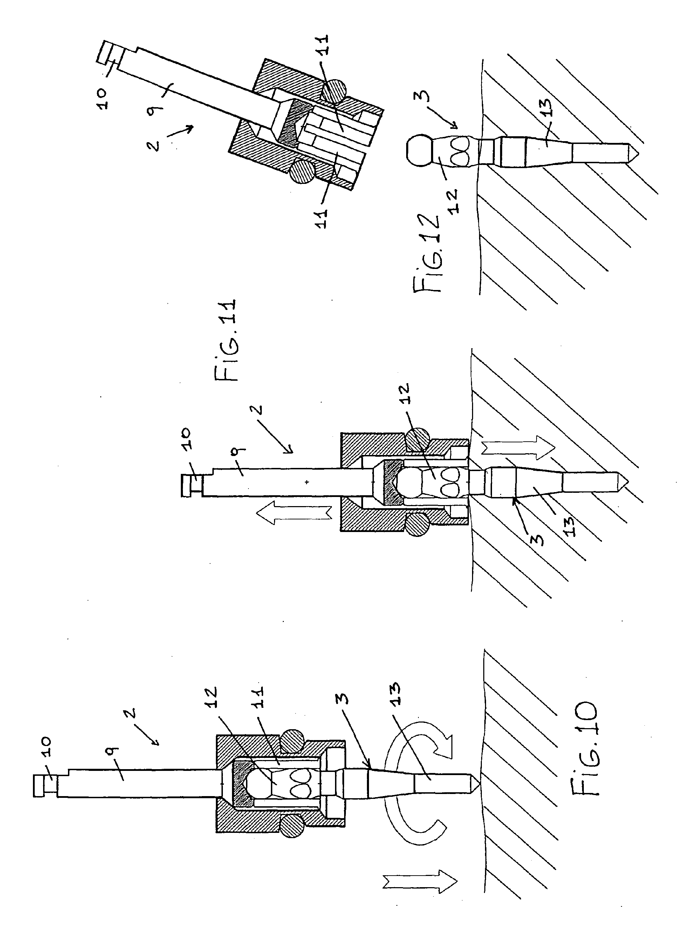

Then, he/she applies the implant at the desired region and drives the electric motor on, thereby screwing the implant in its preset surgical seat or recess, as is schematically in figure 10.

After having achieved a preset depth for the endo-osseous implant, the ring-nut 7 is disengaged from the gripper 8 by automatically stopping, even in a operating or on condition of the micromotor, the rotary movement and accordingly the penetration of the endo-osseous implant 3.

The disengagement of the ring-nut 7 from the gripper occurs because of an axial sliding of the gripper 8 with respect to the ring-nut 7, which disengage the axial legs 11 from the axial inner recess of said ring-nut, by releasing the gripping on the stem 12 of the endo-osseous implant 3 and disengaging it from the rotary gripper.

The axial sliding between the ring-nut 7 and gripper 8 occurs as the ring-nut 7 abuts against the outer surface , whereas the endo-osseous implant 3 is lowered and threaded in the surgical recess, as is clearly shown in figure 11.

Figure 12 shows a following operating step in which the gripper - ring-nut assembly 2 is removed after having set the endo-osseous implant 3 in its proper surgical recess.

It has been found that the invention fully achieves the intended aim and objects.

In fact, the invention provides a device constituting a novel gripping and transporting system for transporting the endo-osseous implant in a sterile blister pack and from the latter to the surgical operating place, in a ready for use condition.

Said device, moreover, allows the implant body to be mechanically automatically threaded by using a micromotor.

The main advantage of the subject device is that it further allows a preset and automatic disengagement of the implant bearer-threader and implant, upon having achieved a proper surgical depth.

Thus, it is possible to provide an automatic surgical and disengagement stop, after having proper applied the implant.

The device according to the invention provides moreover a lot of further very important advantages: in fact, it allows the implant to be properly packaged in a sterile blister pack, while constituting an entrained and rotary key to allow the implant to be easily introduced in the bone and, furthermore, provides a preset and automatic surgical stop.

Thus, after having achieved a preset depth of the endo-osseous implant, the ring-nut is automatically disengaged from the gripper while automatically stopping, even with an operating micromotor continuously on, the rotary movement and accordingly the penetration of the implant into the bone.

In practicing the invention, the used materials, as well as the contingent size and shapes, can be any, depending on requirements.

## Claims

1. A device for packaging and handling an endo-osseous implant, **characterized in that** said device comprises a gripper and ring-nut assembly supporting said endo-osseous implant, which is arranged in a package made rigid with said gripper and ring-nut assembly, and that said gripper and ring-nut assembly coupled to an implant bearer-threader device for mechanically threading said endo-osseous implant in a surgical seat, disengagement means for automatically disengaging said endo-osseous implant from said bearer-threader device after having achieved a set penetration depth being moreover provided.

2. A device according to claim 1, **characterized in that** said package comprises a plastics material cylinder having a closed end portion and including, at the other end portion thereof, an opening allowing said gripper and ring-nut assembly, and said implant to be introduced into said cylinder.

3. A device according to claim 2, **characterized in that** said gripper and ring-nut assembly is tightly coupled to said package through sealing means comprising a O-ring gasket coupled to the ring-nut of said gripper and ring-nut assembly.

4. A device according to claim 3, **characterized in that** said ring-nut comprises an outer annular recess for engaging therein said gasket, and an inner axial recess, for engaging therein a portion of said gripper.

5. A device according to claim 4, **characterized in that** said gripper comprises a gripper stem having an end piece and being rigidly coupled to a gripper portion including a plurality of axially extending gripper legs.

6. A device according to claim 5, **characterized in that** said axially extending gripper legs are designed for engaging a stem of said endo-osseous implant including a threaded portion.

7. A device according to claim 1, **characterized in that** said gripper and ring-nut assembly may be removed from said package by an electric handle tool controlled by a surgical operator.

8. A device according to claim 1, **characterized in that**, upon achieving a preset depth of said endo-osseous implant, said ring-nut is disengaged from said gripper while automatically stopping, even with a micromotor in and on condition, a rotary movement and accordingly a penetration of said implant into a bone.

9. A device according to claim 8, **characterized in that** said ring-nut is disengaged from said gripper by causing said gripper to axially slide with respect to said ring-nut, so as to disengage said axially extending legs from said inner axial recess of said ring-nut, thereby releasing a stem portion of said endo-osseous implant to disengage said endo-osseous system from said gripper.

10. A device according to claim 9, **characterized in that** said axial sliding of said ring-nut and gripper is achieved by causing said ring-nut to abut against an outer surface and lowering said endo-osseous implant by threading it in said surgical seat.
